# EUROPEAN PATENT APPLICATION

(11) **EP 3 841 971 A1**
(43) Date of publication of application: **30.06.2021**
(21) Application number: 19851983.7
(22) Date of filing: 20.08.2019
(51) Int. Cl.: A61B 5/042

(54) **SNARE-INTEGRATED MYOCARDIAL ELECTRICAL SIGNAL-DETECTING CATHETER**

(30) Priority: 21.08.2018 KR 20180097171
(71) Applicant: Taupnu Medical Co., Ltd., Busan 46241 (KR)
(72) Inventor: KIM, June Hong, Busan 48516 (KR); PARK, Kyone Peter, Yangsan-si Gyeongsangnam-do 50653 (KR)
(74) Representative: dompatent von Kreisler Selting Werner - Partnerschaft von Patent- und Rechtsanwälten mbB
(86) International application number: PCT/KR2019/010529
(87) International publication number: WO 2020/040511

(57) **Abstract**

A snare-integrated myocardial electrical signal-detecting catheter is proposed. The snare-integrated myocardial electrical signal-detecting catheter enables a cerclage wire to pass through the His bundle by way of detecting an electrical signal from the myocardium, and safely guides the cerclage wire into a patient's body by capturing the cerclage wire, which has passed through the His bundle, with a snare built in the catheter. The snare-integrated myocardial electrical signal-detecting catheter includes: a catheter having a hollow space to insert a guidewire thereinto, and having a distal part thereof coupled to at least one or more electrodes to detect an electrical signal of the myocardium; a snare lumen built along a longitudinal direction in a sidewall of the catheter, and having a hollow space therein; and a snare inserted into the snare lumen and having one end thereof provided with at least one or more annular wires.

## Description

### Technical Field

The present invention relates to a snare-integrated myocardial electrical signal-detecting catheter and, more particularly, to a snare-integrated myocardial electrical signal-detecting catheter that enables a cerclage wire to pass through the His bundle by way of detecting an electrical signal from the myocardium, and safely guides the cerclage wire into the patient's body by capturing the cerclage wire, which has passed through the His bundle, with a snare built in the catheter.

### Background Art

The cardiac conduction system consists of components conducting signals, starting with the sinoatrial node in the atrium, followed by the atrioventricular node in the atrium and the right bundle and left bundle being branched in the His bundle of the ventricles, and ending with the Purkinje fibers.

In an electrocardiogram, a QRS wave is formed by the depolarization process of ventricular muscle, wherein the first down wave following a P wave is called a Q wave, the first up wave is called an R wave, and the down wave following the R wave is called an S wave. The width of the QRS means the time in which electricity is conducted from the His bundle to the entire ventricle. In the normal state, the time width of the QRS wave is less than about 0.12 seconds (i.e., around 90 ms). When a time width of the QRS wave is 0.12 seconds (i.e., 120 ms) or more, the time width implicates that a ventricular conduction disorder exists. The longer the conduction time, the wider the QRS width, and conversely, the shorter the conduction time, the narrower the QRS width. A wide QRS wave represents ventricular desynchronization in which ventricular movements are not unified, causing a side effect resulting in loss of ventricular function.

When insufficiency occurs in the heart's electrical conduction system itself, or when electricity is abnormally generated outside the conduction system and the electricity is transferred to anywhere else, cardiac arrhythmia occurs.

Methods for diagnosing patients with cardiac arrhythmia include electrocardiography tests, Holter monitoring tests, electrophysiological tests, etc.

Among these methods, the electrophysiological tests measure and record electrical activity in various areas of the heart or observe reaction of the heart by directly applying electrical stimulation to the various areas of the heart, in a state of inserting a thin catheter equipped with electrodes into the heart through a blood vessel of the arm or leg. Through such electrophysiological tests, doctors may pinpoint a causative area where cardiac arrhythmia occurs, perform a procedure to remove the identified causative area or implant an artificial cardiac pacemaker, thereby treating cardiac arrhythmia.

As a part of this research, the present inventor proposed and filed a method, a device, and a catheter in Korean Patent Application Publication No. 10-2016-0011530 and Korean Patent Application Publication No. 10-2016-0020887, wherein a tip of a lead of the artificial cardiac pacemaker that has passed through the coronary sinus is allowed to be positioned in the interventricular septum. The above Korean Patent Application Publications are invented by improving a cerclage wire in Korean Patent Application Publication No. 10-1116867 proposed by the present inventor, and relate to a device capable of fixing an artificial cardiac pacemaker to the heart more conveniently, compared to those devices of the related art.

However, when implanting an artificial cardiac pacemaker to treat cardiac arrhythmia, the electrical conduction time is shortened and a narrow QRS wave may be obtained when electrical stimulation is applied to a position near the conduction system positioned in the interventricular septum. Accordingly, there is a need for research on the device that may be safely and conveniently fixed to the heart and at the same time apply the electrical stimulation to the position near the conduction system.

### Disclosure

### Technical Problem

An objective of the present invention is to provide a snare-integrated myocardial electrical signal-detecting catheter for detecting an electrical signal of the myocardium to identify a position of the His bundle.

In addition, another objective of the present invention is to provide a snare-integrated myocardial electrical signal-detecting catheter provided with a built-in snare for capturing a cerclage wire passing through the His bundle.

The objectives of the present invention are not limited to the objectives mentioned above, and other objectives not mentioned herein will be clearly understood by those skilled in the art from the following description.

### Technical Solution

The present invention to achieve the above objectives relates to a snare-integrated myocardial electrical signal-detecting catheter including: a catheter having a hollow space to insert a guidewire thereinto, and having a distal part thereof coupled to at least one or more electrodes to detect an electrical signal of the myocardium; a snare lumen built along a longitudinal direction in a sidewall of the catheter, and having a hollow space therein; and a snare inserted into the snare lumen and having one end thereof provided with at least one or more annular wires.

The catheter may include a hole formed on one side thereof, the hole may be coupled to and communicates with an end of a distal part of the snare lumen, and the snare may protrude from the hole.

In addition, a controller may be installed at an end of a proximal part of the catheter, and the distal part of the catheter may be controlled to move according to control of the controller.

In addition, the snare may include: a capture part including one or more annular wires; and a body part having one end thereof coupled to the annular wires and having the other end thereof passing through the snare lumen to be positioned outside a patient's body, and the other end of the body part may be pushed or pulled so as to allow the capture part of the snare to be inserted into or protrude from the snare lumen.

In addition, the capture part may be made of a shape memory alloy, or an elastic body, or a self-expanding stent, thereby being folded when inserted into the snare lumen and being unfolded when protruding from the snare lumen.

### Advantageous Effects

The snare-integrated myocardial electrical signal-detecting catheter according to the present invention may detect electrical signals of the myocardium and allow a cerclage wire to pass through a position of the myocardium desired by the user.

In addition, the snare-integrated myocardial electrical signal-detecting catheter according to the present invention may capture the cerclage wire, which has passed through the myocardium, with a built-in snare and safely guide the cerclage wire to outside the patient's body.

In addition, the snare-integrated myocardial electrical signal-detecting catheter according to the present invention may select a snare among various shapes of snares according to the needs of a user, and may be used by inserting the snare into a snare lumen.

### Description of Drawings

FIG. 1 is a perspective view showing a snare-integrated myocardial electrical signal-detecting catheter according to the present invention.
FIG. 2 is a cross-sectional view showing the snare-integrated myocardial electrical signal-detecting catheter according to the present invention.
FIG. 3 is a perspective view showing a snare according to the present invention.
FIG. 4 is a perspective view showing that the snare-integrated myocardial electrical signal-detecting catheter is controlled by using a controller in the present invention.
FIG. 5 is a view showing another exemplary embodiment of a snare lumen according to the present invention.
FIGS. 6a to 6d are views showing a treatment procedure using the snare-integrated myocardial electrical signal-detecting catheter, the treatment procedure being divided into and then illustrated in FIGS. 6a to 6d.

### <Description of the Reference Numerals in the Drawings>

10 : catheter
12 : electrode
14 : hole
20 : snare lumen
30 : snare
32 : capture part
34 : body part
40 : guidewire
50 : controller
60 : cerclage wire

### Best Mode

Benefits and features of the present invention and methods of accomplishing the same may be understood more readily by reference to the following detailed description of exemplary embodiments and the accompanying drawings. However, the present invention is not limited to the exemplary embodiments disclosed below, but will be implemented in a variety of different forms. These exemplary embodiments are provided only to complete the disclosure of the present invention and to completely inform the scope of the present invention to those skilled in the art to which the present invention pertains, and the present invention is only defined by the scope of the claims.

Hereinafter, exemplary embodiments of the present invention will be described in detail with reference to the accompanying drawings. The same reference numerals refer to the same components regardless of the drawings, and "and / or" includes each and every combination of one or more of the mentioned items.

The terminology used herein is for the purpose of describing particular exemplary embodiments only and is not intended to be limiting of the present invention. In this specification, the singular form also includes the plural form unless otherwise specified in the phrase. As used herein, "comprises" and/or "comprising" does not exclude the presence or addition of one or more other components in addition to the mentioned components.

Unless otherwise defined, all terms (including technical and scientific terms) used in the present specification may be used in a sense that can be commonly understood by those skilled in the art. In addition, terms defined in the commonly used dictionary are not ideally or excessively interpreted unless specifically defined.

Hereinafter, preferred exemplary embodiments of the present invention will be described in detail with reference to the accompanying drawings.

In Korean Patent Application Publication No. 10-2016-0011530, the present inventor disclosed "METHOD, DEVICE, AND CATHETER, WHEREIN TIP OF LEAD OF ARTIFICIAL CARDIAC PACEMAKER THAT HAS PASSED THROUGH CORONARY SINUS IS ALLOWED TO BE POSITIONED IN INTERVENTRICULAR SEPTUM". As a method for more effectively transmitting electrical stimulation in a treatment using an artificial cardiac pacemaker of the heart, the above invention is to position a tip of a lead of the artificial cardiac pacemaker that has passed through the coronary sinus in the interventricular septum.

In the above invention, in order to place the tip of the lead of the artificial cardiac pacemaker that has passed through the coronary sinus in the interventricular septum, a surgical wire (i.e., cerclage wire) is inserted into the coronary vein to pass through the interventricular septum, the surgical wire (i.e., cerclage wire) is guided into and fixed in the patient's body, and then the pacemaker lead is inserted into the coronary sinus along the surgical wire (i.e., cerclage wire), thereby positioning the tip of the pacemaker lead in the interventricular septum.

The present invention is an improved invention of a capture catheter previously invented by the present inventor, wherein a position of the His bundle where the conduction system is located in the interventricular septum is identified, a surgical wire (i.e., cerclage wire) is allowed to pass through the His bundle, and a tip of a pacemaker lead is fixed to the His bundle, thereby enabling electrical stimulation to be transmitted more effectively.

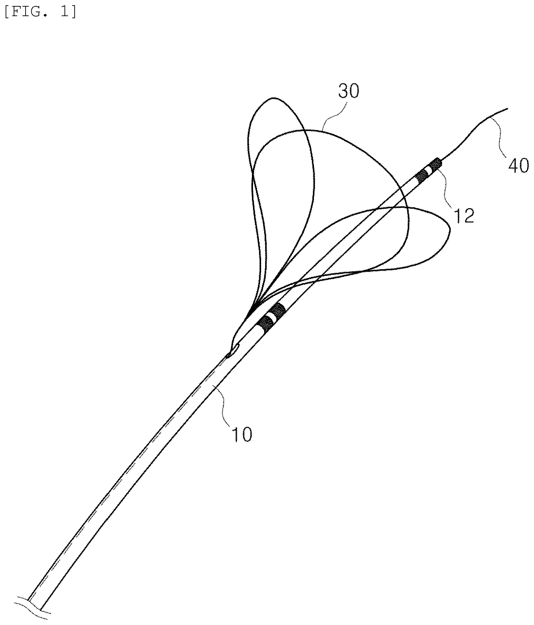

FIG. 1 is a perspective view showing a snare-integrated myocardial electrical signal-detecting catheter according to the present invention.

Referring to FIG. 1, the snare-integrated myocardial electrical signal-detecting catheter according to the present invention includes a catheter 10, a snare lumen 20, and a snare 30.

The catheter 10 has a shape of a long thin hose having a hollow space therein, and may be made of synthetic resin materials, such as soft rubber material and soft plastic material, and is made by using soft and highly ductile material with excellent flexibility and resilience.

A guide wire 40 is inserted into the hollow space of the catheter 10, and the catheter 10 moves into the patient's body along the guidewire 40 inserted first in the patient's body.

A plurality of electrodes 12 is coupled to a distal part of the catheter 10 to detect electrical signals of the myocardium. Accordingly, the catheter 10 may also be referred to as an electrophysiology catheter (EP catheter).

The electrophysiology catheter places an electrode lead in each area of the heart, thereby recording local electrical phenomena, so as to be generally used to determine the characteristics of the patient's general conduction system and be also used to block the causative area of cardiac arrhythmia by transmitting electrical stimulation to the patient's heart.

In general, when performing the catheter treatment, a part positioned relatively far from a surgeon with respect to the surgeon's position is referred to as a distal part, and a part positioned adjacent to the surgeon is referred to as a proximal part.

An electrical signal detected by the electrode 12 may be observed with the naked eye of the surgeon using an external recording device outside the patient's body.

The catheter 10 may not have a hollow space therein, and in the case of an exemplary embodiment in which the interior of the catheter 10 does not have the hollow space therein, the catheter 10 moves into the patient's body without a guidewire 40.

The snare lumen 20 is built in the sidewall of the catheter 10, is formed to be long and thin along the longitudinal direction of the catheter 10, and has a hollow space therein.

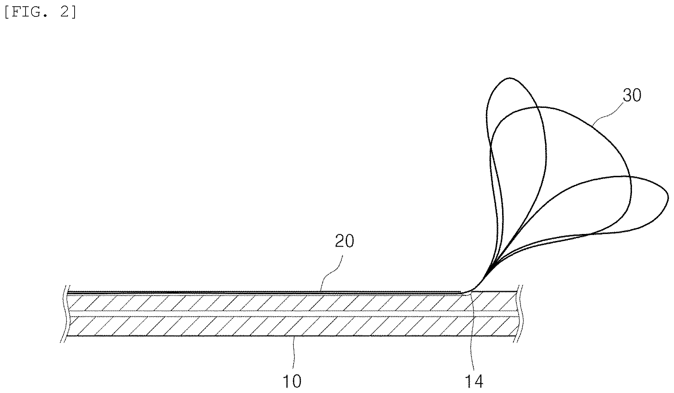

The shape of the snare lumen 20 may be known in more detail with reference to FIG. 2. Characteristically, the outer diameter of the snare lumen 20 is formed to have a size similar to the diameter size of a through hole of the catheter 10, and the snare lumen 20 is made of the same material as the catheter 10, so as to have excellent flexibility and resilience. The snare lumen 20 is preferably built in the inner sidewall of the catheter 10, but is not limited thereto.

Referring to FIG. 2, an end of the distal part of the snare lumen 20 is preferably coupled to and communicated with a hole 14 formed on one side of the catheter 10, but is not limited thereto. Accordingly, when the snare 30 inserted into the snare lumen 20 is pushed toward the distal part, the snare 30 protrudes from the hole 14.

According to a preferred exemplary embodiment of the present invention, the snare 30 is preferably moved into the patient's body in a form inserted into the snare lumen 20, that is, in the form in which the snare 30 is embedded in the catheter 10, but is not limited thereto, and it is also possible for the snare 30 to be inserted into the snare lumen 20 after moving the catheter 10 into the patient's body first.

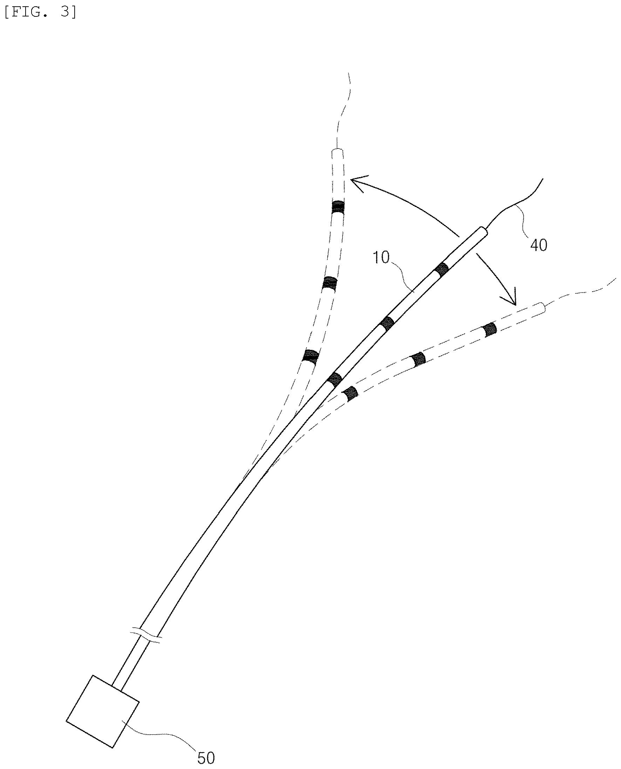

FIG. 3 is a perspective view showing the snare according to the present invention. Referring to FIG. 3, the snare 30 includes: a capture part 32 including one or more annular wires; and a body part 34 having one end thereof coupled to the capture part 32 and having the other end thereof passing through the snare lumen 20 to be positioned outside the patient's body.

The annular wire of the capture part 32 is made of a shape-memory alloy, or an elastic body, or a self-expanding stent, so characteristically, when inserted into the snare lumen 20, the annular wire is folded, and when protruding from the snare lumen 20, the annular wire is unfolded.

Referring to FIG. 3, the snare 30 according to the preferred exemplary embodiment of the present invention shows that the lower end of the capture part 32 having three annular rings is combined, but is not limited thereto, and the capture part 32 may be a single annular ring, or may be a polygonal ring rather than a circular ring.

In addition, the snare 30 according to the preferred exemplary embodiment of the present invention shows that the body part 34 is formed of a single wire, but may be formed by several wires that are twisted with each other, and may be made of a rubber material, or a soft plastic material, or a synthetic resin material, the materials having a shape of an elongated rod with flexibility and good resilience as the catheter 10.

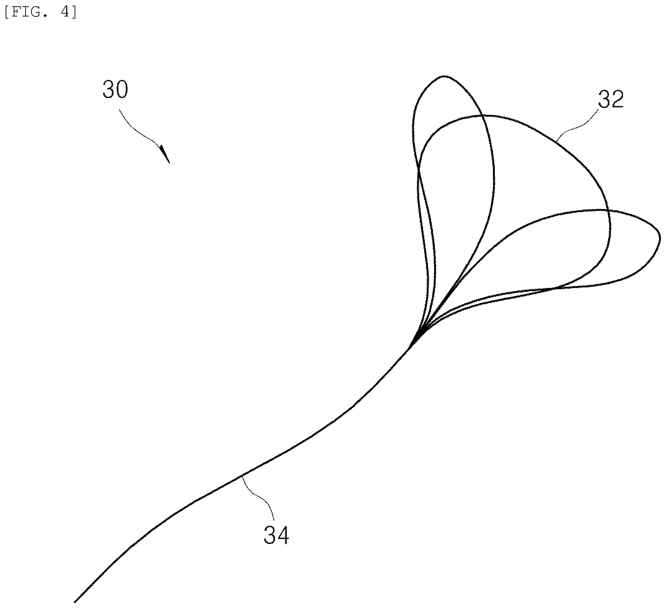

FIG. 4 is a perspective view showing the snare-integrated myocardial electrical signal-detecting catheter according to the present invention controlled by using a controller.

Referring to FIG. 4, a controller 50 is installed at the end of the proximal part of the catheter 10 according to the present invention, and the controller 50 may be operated by a surgeon outside the patient's body. As the controller 50 is controlled, the distal part of the catheter 10 moves left and right, and moves to a specific position of the myocardium desired by the surgeon, so that the electrode coupled to the distal part of the catheter 10 detects the electrical stimulation of the myocardium.

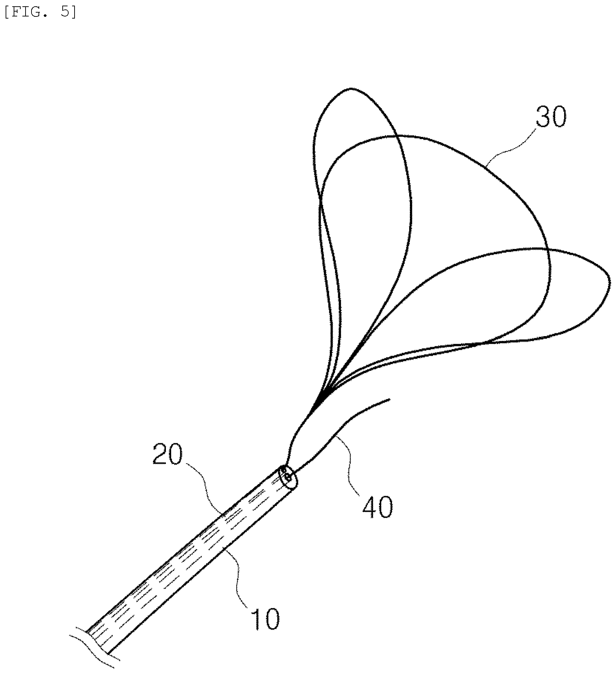

FIG. 5 is a view showing another exemplary embodiment of a snare lumen according to the present invention.

Referring to FIG. 5, in another exemplary embodiment according to the present invention, the snare lumen 20 is built in a sidewall of a catheter 10 along the longitudinal direction to an end of a distal part of the catheter 10, and the snare lumen 20 has a hollow space therein.

Accordingly, when a hole is not formed on one side of the catheter 10 and the snare 30 inserted into the snare lumen 20 is pushed toward the distal part, the snare 30 is pushed to the end of the distal part of the snare lumen 20, the end being positioned identically to the end of the distal part of the catheter 10, whereby the snare 30 protrudes to the outer side of the snare lumen 20.

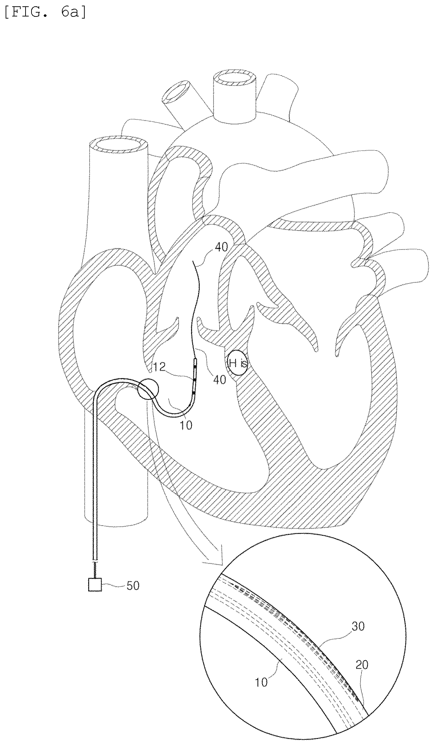

FIGS. 6a to 6d are views showing a treatment procedure using the snare-integrated myocardial electrical signal-detecting catheter, the treatment procedure being divided into and then illustrated in FIGS. 6a to 6d.

FIG. 6a shows that the guidewire 40 is inserted into the hollow space of the catheter 10 according to the present invention, and the snare-integrated myocardial electrical signal-detecting catheter according to the present invention moves along a path where the guidewire 40 is first inserted into the patient's body.

In this case, the snare 30 is inserted into and moved in the snare lumen 20.

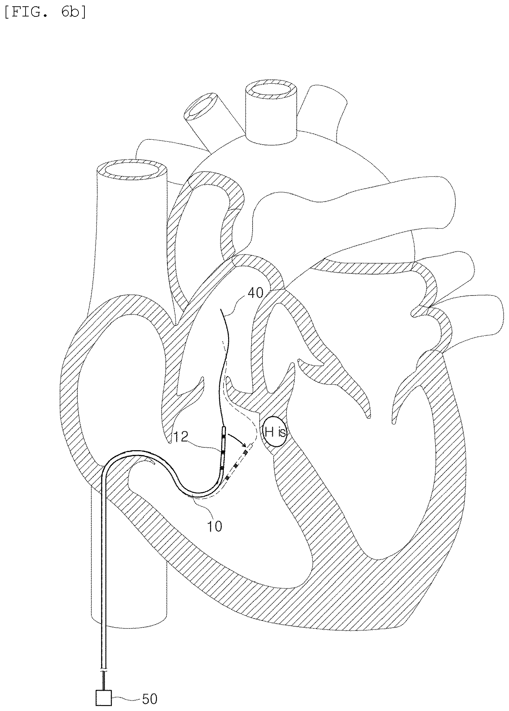

FIG. 6b shows that the controller 50 installed at the end of the proximal part of the catheter 10 detects the His bundle of the interventricular septum by controlling the distal part of the catheter 10 to the left and right. The electrode 12 coupled to the distal part of the catheter 10 detects an electrical signal of the myocardium and finds the position of the His bundle. The His bundle shows an electrocardiogram having a shape different from those of the electrocardiograms showing at other myocardial positions, and the shape may be checked by the surgeon through the external recording device.

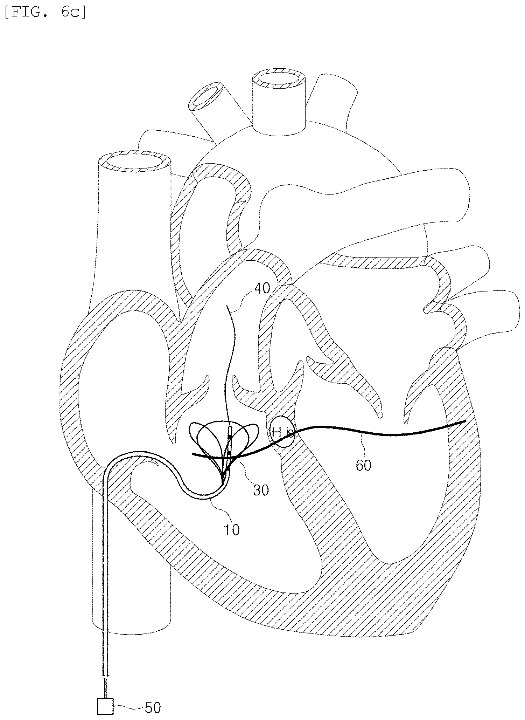

FIG. 6c shows that the cerclage wire 60 is passed through the detected position of the His bundle, and the snare 30 is pushed toward the distal part direction, so that the capture part 32 of the snare 30 protrudes and is unfolded.

After allowing the cerclage wire 60 to pass through the His bundle, the end of the cerclage wire 60 is to be positioned on the capture part 32.

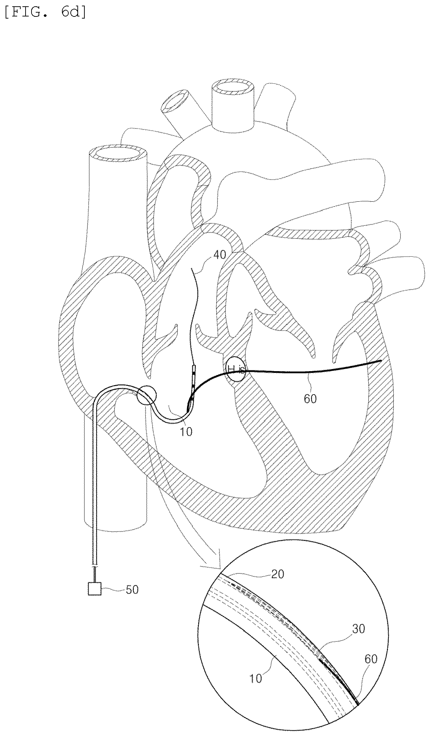

FIG. 6d shows that the snare 30 is pulled so as to capture the cerclage wire 60, and then the snare 30 and the cerclage wire 60 are inserted into the snare lumen 20.

Therefore, when the snare 30 is pulled toward the proximal part, the end of the cerclage wire 60 is captured by the capture part 32 of the snare 30, the snare 30 and the cerclage wire 60 are inserted into the snare lumen 20, and the snare-integrated myocardial electrical signal-detecting catheter is removed from the patient's body, thereby safely guiding the cerclage wire 60 to the outside of the patient's body.

Although the exemplary embodiments of the present invention have been described above with reference to the accompanying drawings, it will be understood that those skilled in the art to which the present invention pertains may implement the present invention in other specific forms without departing from the technical spirit or essential features thereof. Therefore, the exemplary embodiments described above are to be understood in all respects as illustrative and not restrictive.

## Claims

1. A snare-integrated myocardial electrical signal-detecting catheter comprising:
a catheter having a hollow space to insert a guidewire thereinto, and having a distal part thereof coupled to at least one or more electrodes to detect an electrical signal of the myocardium;
a snare lumen built along a longitudinal direction in a sidewall of the catheter, and having a hollow space therein; and
a snare inserted into the snare lumen and having one end thereof provided with at least one or more annular wires.

2. The snare-integrated myocardial electrical signal-detecting catheter of claim 1, wherein the catheter comprises a hole formed on one side thereof,
the hole is coupled to and communicates with an end of a distal part of the snare lumen, and
the snare protrudes from the hole.

3. The snare-integrated myocardial electrical signal-detecting catheter of claim 1, wherein a controller is installed at an end of a proximal part of the catheter, and
the distal part of the catheter is controlled to move according to control of the controller.

4. The snare-integrated myocardial electrical signal-detecting catheter of claim 1, wherein the snare comprises:
a capture part including one or more annular wires; and
a body part having one end thereof coupled to the annular wires and having the other end thereof passing through the snare lumen to be positioned outside a patient's body, and
the other end of the body part is pushed or pulled so as to allow the capture part of the snare to be inserted into or protrude from the snare lumen.

5. The snare-integrated myocardial electrical signal-detecting catheter of claim 4, wherein the capture part is made of a shape memory alloy, or an elastic body, or a self-expanding stent, thereby being folded when inserted into the snare lumen and being unfolded when protruding from the snare lumen.

6. A snare-integrated myocardial electrical signal-detecting catheter comprising:
a catheter having a distal part thereof coupled to at least one or more electrodes to detect an electrical signal of the myocardium;
a snare lumen built along a longitudinal direction in a sidewall of the catheter, and having a hollow space therein; and
a snare inserted into the snare lumen and having one end thereof provided with at least one or more annular wires.

7. The snare-integrated myocardial electrical signal-detecting catheter of claim 6, wherein the catheter comprises a hole formed on one side thereof,
the hole is coupled to and communicates with an end of a distal part of the snare lumen, and
the snare protrudes from the hole.

8. The snare-integrated myocardial electrical signal-detecting catheter of claim 6, wherein a controller is installed at an end of a proximal part of the catheter, and
the distal part of the catheter is controlled to move according to control of the controller.

9. The snare-integrated myocardial electrical signal-detecting catheter of claim 6, wherein the snare comprises:
a capture part including one or more annular wires; and
a body part having one end thereof coupled to the annular wires and having the other end thereof passing through the snare lumen to be positioned outside a patient's body, and
the other end of the body part is pushed or pulled so as to allow the capture part of the snare to be inserted into or protrude from the snare lumen.

10. The snare-integrated myocardial electrical signal-detecting catheter of claim 9, wherein the capture part is made of a shape memory alloy, or an elastic body, or a self-expanding stent, thereby being folded when inserted into the snare lumen and being unfolded when protruding from the snare lumen.
